# FASCICULE DE BREVET EUROPEEN

(11) **EP 0 453 337 B1**
(45) Date de publication et mention de la délivrance du brevet: **01.10.1997**
(21) Numéro de dépôt: 91400743.0
(22) Date de dépôt: 20.03.1991
(51) Int. Cl.: A61K 31/505, A61K 31/71, A61K 31/14, A61K 31/155

(54) **Utilisation de l'héxétidine ou de ses dérivés ou sels pour la préparation d'une composition pharmaceutique à usage ophtalmologique**
Verwendung von Hexetidin oder dessen Derivaten oder Salzen zur Herstellung einer pharmazeutischen Zusammensetzung für ophthalmische Verwendung
Use of hexetidine or its derivatives or salts for the preparation of a pharmaceutical composition for ophthalmic use

(30) Priorité: 18.04.1990 FR 9004945
(43) Date de publication de la demande: 23.10.1991
(73) Titulaire: Salkin, André, F-76520 Boos (FR)
(72) Inventeur: Salkin, André, F-76520 Boos (FR)
(74) Mandataire: Portal, Gérard

(56) Documents cités:
- EP-A- 0 136 231
- GB-A- 793 379
- R.Dolder et coll.(Editeurs) 'Ophthalmika' Wissenschaftliche Verlagsgesellschaft Mbh, Stuttgart, DE, pages 55-67, 92-94, 477-482

## Description

La présente invention concerne essentiellement l'utilisation de l'hexétidine pour aseptiser les prothèses oculaires, en particulier les lentilles de contact.

On connaît par le document EP-B-0 136 231 une composition à pouvoir bactéricide élevé contenant un biguanide et une pyrimidine, en particulier l'hexétidine. L'hexétidine était utilisée pour renforcer le pouvoir fongicide des biguanides. Les utilisations indiquées concernent la désinfection ordinaire de la peau.
R.DOLDER et Coll. (Editeurs), "Ophtalmika", 1983, Wissenschaftliche Verlagsgesellschaft mbH, Stuttgart, DE, pages 477-482, décrit page 480, colonne de gauche sous "Desinfektionslösungen" une composition pour désinfecter les lentilles de contact. Cette composition contient comme produit actif du digluconate de chlorhexidine.
Par ailleurs, on trouve dans la publication de R.DOLDER et Coll. (Editeurs), "Ophtalmika", 1983 , Wissenschaftliche Verlagsgesellschaft mbH, Stuttgart, DE, pages 55-67, la description de bons nombres d'agents antibiotiques utilisés en ophtalmologie et la description de leur mécanisme et étendue d'action.

Le brevet GB-A-793 379 décrit différentes compositions d'hygiène contenant des dérivés de la 5-amino-hexahydropyrimidine. Parmi les compositions citées dans ce document, l'exemple 10 décrit une composition à usage ophtalmique contenant 0,1 % d'hexétidine.

Il vient d'être découvert de manière totalement inattendue que l'hexétidine ou ses dérivés ou sels pouvaient être utilisés pour aseptiser les prothèses de l'oeil ou les lentilles de contact.

Ainsi, la présente invention a pour but de résoudre le nouveau problème technique consistant en la fourniture d'une composition éventuellement pharmaceutique à activité antiseptique destinée à l'aseptisation des prothèses oculaires, et notamment des lentilles de contact, d'une grande efficacité, simple d'emploi et n'ayant pas d'effets nocifs vis-à-vis des tissus de l'oeil, ou vis-à-vis des prothèses de l'oeil ou des lentilles de contact.

Ce problème technique est résolu pour la première fois par la présente invention d'une manière particulièrement simple, peu coûteuse, utilisable à l'échelle industrielle.

Ainsi, la présente invention concerne l'utilisation de l'hexétidine, de ses dérivés ou ses sels, comme principe actif d'une composition éventuellement pharmaceutique à usage antiseptique, destinée à l'aseptisation des prothèses oculaires et notamment des lentilles de contact.

La composition éventuellement pharmaceutique se présente sous forme d'une composition de traitement d'hygiène permettant d'aseptiser les prothèses occulaires ou les lentilles de contact comprenant une concentration en hexétidine ou en ses dérivés ou sels comprise entre 10⁻⁷ % et 0,04 %.

Selon encors une autre variante de réalisation, la composition est formulée sous forme d'une composition liquide, pâteuse, ou sous forme de poudre, ou sous forme d'un support solide imprégné de la composition, ce support est en particulier choisi parmi une compresse, un tissu ou une gaze ou une compresse polyuréthanne.

Selon encore une autre variante de réalisation, la composition est une composition liquide comprenant avantageusement un tampon permettant de régler le pH.

Selon encors une autre variante de réalisation, la composition est une composition liquide comprenant un tensioactif non ionique amphotère, cationique ou anionique, de préférence de type non ionique.

Selon encore une autre variante de réalisation, la composition éventuellement pharmaceutique comprend un agent séquestrant, en particulier de la gluconolactone, en particulier la δ-gluconolactone, ou l'acide éthylènediaminetétraacétique (EDTA).

Selon encore une autre variante de réalisation, la composition pharmaceutique comprend au moins un autre agent antiseptique ou au moins un agent antibiotique.

Selon encore une autre variante de réalisation, l'agent antiseptique précité est choisi parmi un biguanide, en particulier le polyhexaméthylènebiguanide et ses sels, ou un composé d'ammonium quaternaire et plus particulièrement le chlorure de didécyldiméthylammonium.

Selon encore une autre variante de réalisation, la composition éventuellement pharmaceutique comprend à titre d'agent antibiotique l'érythromycine ou la néomycine.

L'invention concerne encore des compositions éventuellement pharmaceutiques antiseptiques ophtalmiques contenant comme principe actif l'hexétidine, ses dérivés ou ses sels. Des variantes de réalisation particulières de ces compositions résultent de la description précédente relativement à l'utilisation.

Selon un troisième aspect, la présente invention couvre également un procédé de fabrication de compositions pharmaceutiques antiseptiques ophtalmiques, caractérisé en ce qu'il comprend l'incorporation de l'hexétidine de ses dérivés ou sels, en une quantité antiseptiquement efficace, dans un support, véhicule ou excipient ophtalmologiquement acceptable.

Des variantes de réalisation de ce procédé de préparation résultent également de la description précédents relative à l'utilisation.

Grâce à l'invention, on peut réaliser un traitement thérapeutique de tissus sensibles, en particulier des tissus occulaires en l'absence ou en présence de prothèses provisoires ou permanentes de l'oeil, et également en l'absence ou en présence de lentilles de contact, ainsi que le traitement de ces prothéses provisoires ou permanentes et de ces lentilles de contact. L'invention fournit donc des compositions pharmaceutiques ophtalmologiques à pouvoir antiseptique améliorant le confort dans les traitements ophtalmologiques avec une notable diminution de la contamination à gram+/gram- et une diminution de la sensation de brûlure, une amélioration de la cicatrisation des tissus lésés.

Les compositions ophtalmologiques selon l'invention peuvent être formulées sous forme de préparation mère concentrée ou de produits prêts à l'emploi.

L'invention permet de réaliser ces traitements efficaces sans nécessiter la présence partielle ou totale de corticoïdes bien que leur utilisation soit quand même possible. En outre, l'hexétidine, ses dérivés ou ses sels ne modifient pas les matériaux des prothéses ou notamment des lentilles de contact, tandis que les molécules d'hexétidine de ses dérivés ou sels ne s'accumulent également pas dans les prothèses ou les lentilles de contact.

La présente invention va être maintenant décrite relativement à divers exemples démontrant l'efficacité et l'innocuité de l'hexétidine, sous diverses formulations.

### Exemple 1

Composition ophtalmologique contenant de l'hexétidine comme principe actif pour le traitement de tissus conjonctifs ophtalmiques contaminés.

On prépare 5 compositions dénommées A, B, C, D et E, contenant une concentration variable d'hexétidine. L'hexétidine est introduite dans un milieu aqueux à viscosité égale à l'eau, réglée à un pH légèrement acide. Ces préparations peuvent être formulées sous des formes physiques différentes, par exemple sous forme de gel, de crème, de produit pulvérulent ou sur des supports tels que des compresses, tissus ou compresses gazes ou comme compresses polyuréthannes.

| | A | B | C | D | E |
|---|---|---|---|---|---|
| Hexétidine | 0,3 | 0,03 | 0,003 | 0,0003 | 0,00003 |
| H₂O stérile | QSP 100 | QSP 100 | QSP 100 | QSP 100 | QSP 100 |
| Réglage pH avec acide citrique | 6,5 | 6,5 | 6,5 | 6,5 | 6,5 |

On peut aussi régler le pH avec un tampon comprenant de la gluconolactone, de l'acide gluconique, ou de l'acide borique, de l'acide acétique, et un séquestrant comme l'éthylènediaminetétraacétique.

Les solutions A, B, C, D et E ci-dessus ont été testées sur des sujets comportant une inflammation de l'oeil. Les traitements proposés ont été les suivants :
A - 1 goutte une fois par jour,
B - 5 gouttes deux fois par jour,
C - 10 gouttes trois fois par jour,
D - 15 gouttes trois fois par jour,
E - 20 gouttes trois fois par jour.

Dans tous les cas, il a été observé une rapide amélioration et constaté une guérison totale au terme d'une semaine de traitement.

Le traitement a été reproduit sur des sujets portant des lentilles de contact. Le traitement est effectué avant la pose des lentilles de contact sur l'oeil, les lentilles de contact sont portées toute la journée en présence de la composition de traitement selon l'invention.

Les lentilles de contact ont fait l'objet d'un contrôle spectrophotométrique après chaque journée.

Un contrôle visuel de l'aspect de la lentille a été effectué avant le contrôle spectrophotométrique.

Aucune modification d'apparence n'a été observée tant sur les lentilles à 40 % d'hydrométrie (lentille semi-rigide) que sur les lentilles à 70 % d'hydrométrie (lentille souple).

La spectrophotométrie n'a révélé aucune accumulation d'hexétidine.

Avant chaque opération de contrôle, les lentilles ont été rincées à l'aide d'une solution saline dont la réflexion spectrophotométrique est de 95 %.

Les lentilles ont restitué au cours de toute la période du test une spectrophotométrie équivalente variant de 92 à 95.

Les lentilles de contact ayant été trempées dans une solution d'hexétidine du type C chaque nuit, après rinçage dans une solution saline, reproduisaient une réflexion spectrophotométrique équivalente variant de 92 à 95.

L'étude d'absorption d'un produit de traitement ophtalmique à base d'hexétidine selon l'invention, en présence de lentilles de contact et dont ces mêmes lentilles de contact sont mises à tremper dans une solution antiseptique à base d'hexétidine selon l'invention, a démontré une non-absorption de la molécule d'hexétidine dans les matériaux.

Ceci s'applique quelle que soit la nature des lentilles de contact, qu'elles soient semi-rigides ou souple, même après 40 nuits de trempage et 40 jours de port de la lentille.

### Exemple 2

Dans cet exemple, les compositions A, B, C, D et E précédentes de l'exemple 1 sont complétées par ajout d'un agent tensioactif non ionique connu sous l'appellation commerciale "Pluriol F87) à raison de 2 % dans chaque formulation, en donnant ainsi des compositions référencées respectivement A₁, B₁, C₁, D₁ et E₁.

Au terme de la même période d'essai comprenant 40 nuits de trempage des lentilles de contact dans les solutions respectives, suivi d'un rinçage journalier ainsi que d'un port de la lentille pendant le jour, il a pu être observé une non-absorption de la molécule d'hexétidine ainsi que de l'agent tensioactif et non-modification du matériau de la lentille.

### Exemple 3

Les compositions A₁, B₁, C₁, D₁ et E₁ ont été modifiées en les complétant par ajout de 65 mg d'acide éthylènediaminetétraacétique (EDTA) pour 100 g de la composition, en obtenant ainsi des compositions A₂, B₂, C₂, D₂ et E₂.

Les mêmes essais que ceux rapportés à l'exemple 2 ont permis de constater une non-absorption de la molécule d'hexétidine, de l'agent tensioactif ainsi que de l'EDTA, en démontrant ainsi l'innocuité de la composition ainsi formée vis-à-vis des matériaux de la lentille, et ainsi que des tissus occulaires.

### Exemple 4

On prépare diverses compositions A₄, B₄, C₄, D₄ et E₄ à partir des formules A₃, B₃, C₃, D₃ et E₃ de l'exemple 3, par l' ajout de quantités variables de polyhexaméthylènebiguanide (PHMB), comme suit :

| | A₄ | B₄ | C₄ | D₄ | E₄ |
|---|---|---|---|---|---|
| Hexétidine | 0,03 | 0,003 | 0,0003 | 0,00003 | 0,000003 |
| EDTA | 65 mg% | 65 mg% | 65 mg% | 65 mg% | 65 mg% |
| Pluriol F87 | 2 g% | 2 g% | 2 g% | 2 g% | 2 g% |
| PHMB | 0,05 | 0,005 | 0,0005 | 0,00005 | 0,000005 |
| PHMB = polyhexaméthylènebiguanide Réglage pH à 6,5 avec acide citrique ou tampon borique QSP 100 g | | | | | |

Les essais du premier jour par trempage ont montré une absorption du PHMB pour la formule n^{o} A₄ ayant la concentration la plus forte.

Toutes les formulations ont donc été vérifiées et après la période d'essai de 40 jours, on a observé une légère absorption du PHMB pour la formulation B₄ et la non-absorption en spectrophotométrie du PHMB des formules C₄, D₄ et E₄.

Ainsi, en respectant une proportion d'incorporation du PHMB conforme aux formulations C₄ à E₄, on obtient une composition thérapeutiquement efficace n'ayant pas d'effet nocif ni vis-à-vis des tissus occulaires, ni vis-à-vis des matériaux des lentilles.

## Revendications (Revendications pour l'(les) Etat(s) contractant(s) suivant(s): DE, GB, FR, IT, NL, SE, CH, LI, BE, AT, LU, DK)

1. Procédé pour l'aseptisation des prothèses ophtalmiques et des lentilles de contact caractérisé en ce qu'il consiste à traiter ladite prothèse ou ladite lentille par une composition éventuellement pharmaceutique, contenant comme matière active de l'héxétidine ou un de ses sels non toxiques.

2. Procédé selon la revendication 1, caractérisé en ce que la composition se présente sous forme d'une composition dont la concentration en héxétidine ou en ses sels est comprise entre 10⁻⁷ % et 0,04 %.

3. Procédé selon l'une des revendications 1 ou 2, caractérisé en ce que la composition est une composition liquide comprenant un tampon permettant de régler le pH.

4. Procédé selon l'une des revendications 1 à 3, caractérisé en ce que la composition est une composition liquide comprenant un tensioactif non ionique amphotère, cationique ou anionique, de préférence de type non ionique.

5. Procédé selon l'une quelconque des revendications 1 à 4, caractérisé en ce que la composition comprend un agent séquestrant, en particulier de la gluconolactone, en particulier la δ-gluconolactone, ou l'éthylènediaminetétraacétique (EDTA).

6. Procédé selon l'une quelconque des revendications 1 à 5, caractérise en ce que la composition comprend au moins un autre agent antiseptique ou au moins un agent antibiotique.

7. Procédé selon la revendication 6, caractérisé en ce que l'agent antiseptique précité est choisi parmi un biguanide, en particulier le polyhexaméthylènebiguanide et ses sels, ou un composé d'ammonium quaternaire et plus particulièrement le chlorure de didécyldiméthylammonium.

8. Procédé selon l'une des revendications 6 ou 7, caractérisé en ce que la composition comprend à titre d'agent antibiotique l'érythromycine ou la néomycine.

9. Utilisation comme agent destiné à l'aseptisation des prothèses ophtalmiques et des lentilles de contact, d'une composition telle que définie dans l'une des revendications 1 à 8.

10. Utilisation de l'héxétidine ou de ses sels non toxiques pour la préparation d'une composition pharmaceutique à activité antiseptique, telle que définie dans l'une des revendications 1 à 8, destinée à l'aseptisation des prothèses ophtalmiques et des lentilles de contact.

## Revendications (Revendications pour l'(les) Etat(s) contractant(s) suivant(s): ES, GR)

1. Procédé pour l'aseptisation des prothèses ophtalmiques et des lentilles de contact caractérisé en ce qu'il consiste à traiter ladite prothèse ou ladite lentille par une composition éventuellement pharmaceutique, contenant comme matière active de l'héxétidine ou un de ses sels non toxiques.

2. Procédé selon la revendication 1, caractérisé en ce que la composition se présente sous forme d'une composition dont la concentration en héxétidine ou en ses sels est comprise entre 10⁻⁷ % et 0,04 %.

3. Procédé selon l'une des revendications 1 ou 2, caractérisé en ce que la composition est une composition liquide comprenant un tampon permettant de régler le pH.

4. Procédé selon l'une des revendications 1 à 3, caractérisé en ce que la composition est une composition liquide comprenant un tensioactif non ionique amphotère, cationique ou anionique, de préférence de type non ionique.

5. Procédé selon l'une quelconque des revendications 1 à 4, caractérisé en ce que la composition comprend un agent séquestrant, en particulier de la gluconolactone, en particulier la δ-gluconolactone, ou l'éthylènediaminetétraacétique (EDTA).

6. Procédé selon l'une quelconque des revendications 1 à 5, caractérise en ce que la composition comprend au moins un autre agent antiseptique ou au moins un agent antibiotique.

7. Procédé selon la revendication 6, caractérisé en ce que l'agent antiseptique précité est choisi parmi un biguanide, en particulier le polyhexaméthylènebiguanide et ses sels, ou un composé d'ammonium quaternaire et plus particulièrement le chlorure de didécyldiméthylammonium.

8. Procédé selon l'une des revendications 6 ou 7, caractérisé en ce que la composition comprend à titre d'agent antibiotique l'érythromycine ou la néomycine.

9. Utilisation comme agent destiné à l'aseptisation des prothèses ophtalmiques et des lentilles de contact, d'une composition telle que définie dans l'une des revendications 1 à 8.

## Claims (Claims for the following Contracting State(s): DE, GB, FR, IT, NL, SE, CH, LI, BE, AT, LU, DK)

1. Process for asepticising ocular prostheses and contact lenses, characterized in that it consists in treating said prosthesis or said lens by an optionally pharmaceutical composition which comprises, as active material, hexetidine or one of its non-toxic salts.

2. Process according to claim 1, characterized in that the composition is in the form of a composition containing a concentration of hexetidine or its salts of between 10⁻⁷% and 0.04%.

3. Process according to one of claims 1 or 2, characterized in that the composition is a liquid composition which comprises a buffer for adjusting the pH.

4. Process according to one of claims 1 to 3, characterized in that the composition is a liquid composition which comprises a non-ionic, amphoteric, cationic or anionic surfactant, preferably a surfactant of the non-ionic type.

5. Process according to any one of claims 1 to 4, characterized in that the composition comprises a sequestering agent, especially gluconolactone, in particular δ-gluconolactone, or ethylenediaminetetraacetic (EDTA).

6. Process according to any one of claims 1 to 5, characterized in that the composition comprises at least one other antiseptic or at least one antibiotic.

7. Process according to claim 6, characterized in that the aforesaid antiseptic is selected from a biguanide, in particular polyhexamethylenebiguanide and its salts, or a quaternary ammonium compound, more particularly didecyldimethylammonium chloride.

8. Process according to one of claims 6 or 7, characterized in that the composition contains erythromycin or neomycin as the antibiotic.

9. Use as an agent intended for asepticising ocular prostheses and contact lenses, of a composition as defined in one of claims 1 to 8.

10. Use of hexetidine or of its non-toxic salts for the preparation of a a pharmaceutical composition with antiseptic activity, as defined in one of claims 1 to 8, intended for asepticising ocular prostheses and contact lenses.

## Claims (Claims for the following Contracting State(s): ES, GR)

1. Process for asepticising ocular prostheses and contact lenses, characterized in that it consists in treating said prosthesis or said lens by an optionally pharmaceutical composition which comprises, as active material, hexetidine or one of its non-toxic salts.

2. Process according to claim 1, characterized in that the composition is in the form of a composition containing a concentration of hexetidine or its salts of between 10⁻⁷% and 0.04%.

3. Process according to one of claims 1 or 2, characterized in that the composition is a liquid composition which comprises a buffer for adjusting the pH.

4. Process according to one of claims 1 to 3, characterized in that the composition is a liquid composition which comprises a non-ionic, amphoteric, cationic or anionic surfactant, preferably a surfactant of the non-ionic type.

5. Process according to any one of claims 1 to 4, characterized in that the composition comprises a sequestering agent, especially gluconolactone, in particular δ-gluconolactone, or ethylenediaminetetraacetic (EDTA).

6. Process according to any one of claims 1 to 5, characterized in that the composition comprises at least one other antiseptic or at least one antibiotic.

7. Process according to claim 6, characterized in that the aforesaid antiseptic is selected from a biguanide, in particular polyhexamethylenebiguanide and its salts, or a quaternary ammonium compound, more particularly didecyldimethylammonium chloride.

8. Process according to one of claims 6 or 7, characterized in that the composition contains erythromycin or neomycin as the antibiotic.

9. Use as an agent intended for asepticising ocular prostheses and contact lenses, of a composition as defined in one of claims 1 to 8.

## Patentansprüche (Patentansprüche für folgende(n) Vertragsstaat(en): DE, GB, FR, IT, NL, SE, CH, LI, BE, AT, LU, DK)

1. Verfahren, um Augenprothesen und Kontaktlinsen aseptisch zu machen, dadurch gekennzeichnet, daß es aus der Behandlung der Prothese oder der Linse mit einer gegebenenfalls pharmazeutischen Zusammensetzung besteht, die als aktiven Stoff Hexetidin oder eines seiner nicht-toxischen Salze enthält.

2. Verfahren nach Anspruch 1, dadurch gekennzeichnet, daß die Zusammensetzung in Form einer Zusammensetzung dargereicht wird, deren Konzentration von Hexetidin oder einem seiner Salze zwischen 10⁻⁷ % und 0,04 % liegt.

3. Verfahren nach einem der Ansprüche 1 oder 2, dadurch gekennzeichnet, daß die Zusammensetzung eine flüssige Zusammensetzung ist, die einen Puffer umfaßt, der die Regulierung des pH ermöglicht.

4. Verfahren nach einem der Ansprüche 1 bis 3, dadurch gekennzeichnet, daß die Zusammensetzung eine flüssige Zusammensetzung ist, die einen nicht-ionischen, amphoteren, kationischen oder anionischen grenzflächenaktiven Stoff, vorzugsweise vom nichtionischen Typ, umfaßt.

5. Verfahren nach einem der Ansprüche 1 bis 4, dadurch gekennzeichnet, daß die Zusammensetzung ein Sequestrierungsmittel, insbesondere Gluconolacton, insbesondere δ-Gluconolacton, oder Ethylendiamintetraessigsäure (EDTA), umfaßt.

6. Verfahren nach einem der Ansprüche 1 bis 5, dadurch gekennzeichnet, daß die Zusammensetzung zumindest ein weiteres Antiseptikum oder zumindest ein Antibiotikum umfaßt.

7. Verfahren nach Anspruch 6, dadurch gekennzeichnet, daß das Antiseptikum ausgewählt wird aus Biguanid, insbesondere Polyhexamethylenbiguanid und seinen Salzen, oder einer quaternären Ammonium-Verbindung, und insbesondere Didecyldimethylammoniumchlorid.

8. Verfahren nach einem der Ansprüche 6 oder 7, dadurch gekennzeichnet, daß die Zusammensetzung als Antibiotikum Erythromycin oder Neomycin umfaßt.

9. Verwendung einer Zusammensetzung, wie in einem der Ansprüche 1 bis 8 definiert, als Mittel, das dazu bestimmt ist, Augenprothesen und Kontaktlinsen aseptisch zu machen.

10. Verwendung von Hexetidin oder einem seiner nicht-toxischen Salze bei der Herstellung einer pharmazeutischen Zusammensetzung mit antiseptischer Wirksamkeit, wie in einem der Ansprüche 1 bis 8 definiert, die dazu bestimmt ist, Augenprothesen und Kontaktlinsen aseptisch zu machen.

## Patentansprüche (Patentansprüche für folgende(n) Vertragsstaat(en): ES, GR)

1. Verfahren, um Augenprothesen und Kontaktlinsen aseptisch zu machen, dadurch gekennzeichnet, daß es aus der Behandlung der Prothese oder der Linse mit einer gegebenenfalls pharmazeutischen Zusammensetzung besteht, die als aktiven Stoff Hexetidin oder eines seiner nicht-toxischen Salze enthält.

2. Verfahren nach Anspruch 1, dadurch gekennzeichnet, daß die Zusammensetzung in Form einer Zusammensetzung dargereicht wird, deren Konzentration von Hexetidin oder einem seiner Salze zwischen 10⁻⁷ % und 0,04 % liegt.

3. Verfahren nach einem der Ansprüche 1 oder 2, dadurch gekennzeichnet, daß die Zusammensetzung eine flüssige Zusammensetzung ist, die einen Puffer umfaßt, der die Regulierung des pH ermöglicht.

4. Verfahren nach einem der Ansprüche 1 bis 3, dadurch gekennzeichnet, daß die Zusammensetzung eine flüssige Zusammensetzung ist, die einen nicht-ionischen, amphoteren, kationischen oder anionischen grenzflächenaktiven Stoff, vorzugsweise vom nichtionischen Typ, umfaßt.

5. Verfahren nach einem der Ansprüche 1 bis 4, dadurch gekennzeichnet, daß die Zusammensetzung ein Sequestrierungsmittel, insbesondere Gluconolacton, insbesondere δ-Gluconolacton, oder Ethylendiamintetraessigsäure (EDTA), umfaßt.

6. Verfahren nach einem der Ansprüche 1 bis 5, dadurch gekennzeichnet, daß die Zusammensetzung zumindest ein weiteres Antiseptikum oder zumindest ein Antibiotikum umfaßt.

7. Verfahren nach Anspruch 6, dadurch gekennzeichnet, daß das Antiseptikum ausgewählt wird aus Biguanid, insbesondere Polyhexamethylenbiguanid und seinen Salzen, oder einer quaternären Ammonium-Verbindung, und insbesondere Didecyldimethylammoniumchlorid.

8. Verfahren nach einem der Ansprüche 6 oder 7, dadurch gekennzeichnet, daß die Zusammensetzung als Antibiotikum Erythromycin oder Neomycin umfaßt.

9. Verwendung einer Zusammensetzung, wie in einem der Ansprüche 1 bis 8 definiert, als Mittel, das dazu bestimmt ist, Augenprothesen und Kontaktlinsen aseptisch zu machen.
